# EUROPÄISCHE PATENTSCHRIFT

(11) **EP 3 391 255 B1**
(45) Veröffentlichungstag und Bekanntmachung des Hinweises auf die Patenterteilung: **15.01.2025**
(21) Anmeldenummer: 16808664.3
(22) Anmeldetag: 08.12.2016
(51) Int. Cl.: G16H 40/63

(54) **SYSTEM UND VERFAHREN ZUR ERKENNUNG EINES BETRIEBSZUSTANDES ODER EINES BEHANDLUNGSVERLAUFS BEI EINER BLUTBEHANDLUNG**
SYSTEM AND METHOD FOR DETECTING AN OPERATING STATE OR A COURSE OF TREATMENT DURING A BLOOD TREATMENT
SYSTÈME ET PROCÉDÉ DE RECONNAISSANCE D'UN ÉTAT DE FONCTIONNEMENT OU D'UNE PHASE DE TRAITEMENT AU COURS D'UN TRAITEMENT DE SANG

(30) Priorität: 15.12.2015 DE 102015016271
(43) Veröffentlichungstag der Anmeldung: 24.10.2018
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE)
(74) Vertreter: Rau, Jenspeter
(86) Internationale Anmeldenummer: PCT/EP2016/080294
(87) Internationale Veröffentlichungsnummer: WO 2017/102553

(56) Entgegenhaltungen:
- EP-A1- 2 597 584
- DE-A1- 102004 011 264
- US-A1- 2006 289 342
- US-A1- 2011 087 499
- US-A1- 2011 105 979

## Beschreibung

### Technisches Gebiet

Die Erfindung betrifft ein Blutbehandlungssystem und ein computer-implementiertes Verfahren zur Erkennung eines Betriebszustandes oder eines Behandlungsverlaufs oder einer Abweichung von einem idealen oder komplikationsfreien Betriebszustand oder Behandlungsverlauf bei einer Blutbehandlung, insbesondere bei einer extrakorporalen Blutbehandlung.

### Hintergrund

Zur Entfernung von harnpflichtigen Substanzen und zum Flüssigkeitsentzug werden beim chronischen Nierenversagen verschiedene Verfahren zur apparativen Blutbehandlung eingesetzt. Bei der Hämodialyse überwiegt der diffusive Stofftransport über die semipermeable Membran eines Filters, während bei der Hämofiltration ein konvektiver Stofftransport über die Filtermembran stattfindet. Eine Kombination aus beiden Verfahren ist die Hämodiafiltration. Wenn im Folgenden von einer Dialyse die Rede ist, soll dieser Begriff sowohl eine reine Dialysebehandlung als auch eine Hämodiafiltration umfassen.

Ein weiteres Blutbehandlungsverfahren zur Entfernung unerwünschter Substanzen aus dem Blut ist die therapeutische Apherese, bei der eine Trennung von Vollblut und Plasma vorgenommen wird.

Die bekannten Vorrichtungen zur Durchführung der genannten Blutbehandlungsverfahren weisen einen extrakorporalen Blutkreislauf und eine Blutbehandlungseinheit auf. Der extrakorporale Blutkreislauf umfasst eine arterielle Blutleitung, die dem Patienten entnommenes Blut einer Blutbehandlungseinheit zuführt und eine venöse Blutleitung, die behandeltes Blut von der Blutbehandlungseinheit wegführt.

Zu behandelndes Blut wird dem Patienten über einen Patientenzugang entnommen und behandeltes Blut wird darüber auch wieder an den Patienten zurückgegeben.

Bei der Dialyse ist die Blutbehandlungseinheit als Filtereinheit mit einer Blutkammer ausgebildet, die durch eine semipermeable Membran mit der Dialysierflüssigkeitskammer verbunden ist. Die Dialysierflüssigkeitskammer ist mit dem Dialysierflüssigkeitssystem verbunden. Das Dialysierflüssigkeitssystem umfasst eine Dialysierflüssigkeitszuführleitung, die Frischdialysat zur Dialysierflüssigkeitskammer führt sowie eine von der Dialysierflüssigkeitskammer abgehende Dialysierflüssigkeitsabführleitung.

Zur Überwachung eines komplikationsfreien Verlaufs der Dialysebehandlung ist eine Dialysemaschine typischerweise mit einer Vielzahl von Sensoren ausgerüstet. So sind in der Dialysierflüssigkeitszuführleitung und in der Dialysierflüssigkeitsabführleitung jeweils Leitfähigkeitssensoren zur Messung der elektrischen Leitfähigkeit des Dialysats vorgesehen. Ein Vergleich der mit den beiden Sensoren gemessenen Leitfähigkeiten erlaubt einen Rückschluss auf die Reinigungsleistung (Clearance).

In dem extrakorporalen Blutkreislauf sind typischerweise Bluttemperatursensoren Blutdrucksensoren in der arteriellen sowie in der venösen Blutleitung vorgesehen.

Eine automatische Analyse der Sensorsignale beruht typischerweise auf der Über- oder Unterschreitung von Schwellwerten, die zur Überwachung der Sensorsignale eingestellt werden. Die Einstellung der Schwellwerte erfordert einen erfahrenen Bediener und ist häufig fehleranfällig.

Die Durchführung der Blutbehandlung durch das betreuende medizinische Personal beruht zu einem großen Teil auf festgelegten Routinen. Die Interpretation der komplexen Sensorsignale und deren Trendverläufe durch den Anwender kann eine Herausforderung für den Anwender darstellen, wenn es sich um eine untypische Situation handelt. Die Möglichkeit einer daraus resultierenden fehlerhaften Anwenderreaktion stellt ein Risiko für den Patienten dar.

Dokument US2011105979A1 beschreibt Vorrichtungen und Verfahren für die Gesundheitsversorgung, die subjektive und objektive Messwerte über den Gesundheitszustand und die Behandlung des Patienten erfassen. Die Messwerte werden analysiert (z. B. indem sie miteinander und/oder mit Normen korreliert werden) und Berichte erstellt, um die laufende Diagnose und Behandlung des Patienten zu unterstützen sowie Ärzte, Krankenschwestern und andere Pflegekräfte bei der Entscheidungsfindung, der Überwachung der Einhaltung von Behandlungsvorschriften, der Erleichterung der Einhaltung von regulativen Vorgaben, der Rechnungsstellung usw. zu unterstützen. Ein Prozessor generiert Patientenanfragen in Verbindung mit Behandlungen, die von dem Dialysegerät durchgeführt werden. Die Abfragen beziehen sich zumindest teilweise auf subjektive Themen, wie z. B. den Zustand der psychischen Gesundheit und des Wohlbefindens des Patienten, die Lebensqualität, das Ausmaß der Schmerzen, die Einschätzung des Therapieerfolgs.

Dokument US2011087499A1 beschreibt ein System, ein Verfahren und ein computerlesbares Medium für die Planung und Optimierung von Dialysebehandlungen. Das System kann einen Prozessor enthalten, der so programmiert ist, dass er Operationen durchführt, einschließlich des Empfangs eines Dialysebehandlungsparametersatzes und des Abrufs einer vorbestimmten Liste von Dialysatoren und entsprechender Kosten- und Leistungsdaten für jeden Dialysator, wobei die Kostenauswirkung sowohl auf der Grundlage einer klinischen Auswirkung als auch auf der Grundlage eines Kostenwertes bestimmt werden.

Es ist daher die Aufgabe der vorliegenden Erfindung, mindestens eines der angegebenen Probleme zu lösen und ein computergestütztes System anzugeben, dass eine zuverlässige Erkennung eines Betriebszustands oder eines Behandlungsverlaufs einer Blutbehandlung ermöglicht.

### Zusammenfassung

Diese Aufgabe wird gelöst durch, ein Blutbehandlungssystem nach Anspruch 1, ein computerimplementiertes Verfahren nach Anspruch 6, ein Computerprogrammprodukt nach Anspruch 11, sowie ein Computerprogram nach Anspruch 127. Vorteilhafte Ausführungsformen sind in den abhängigen Ansprüchen angegeben.

Eine Ausführungsform betrifft ein Computersystem zur Erkennung eines Betriebszustandes oder eines Behandlungsverlaufs oder einer Abweichung von einem idealen oder komplikationsfreien Betriebszustand einer Blutbehandlungsmaschine oder eines Behandlungsverlaufs einer Blutbehandlung. Beispiele für eine Blutbehandlungsmaschine sind eine Plasmapheresemaschine, eine Hämodialysemaschine, oder eine Hämofiltrationsmaschine.

Das Computersystem umfasst eine erste Schnittstelle, die angepasst ist, ein Betriebsparameterüberwachungssignal zu empfangen, wobei das Betriebsparameterüberwachungssignal ein zeitabhängiges Signal eines Sensors zur Überwachung eines Betriebsparameters der Blutbehandlungsmaschine oder ein von dem zeitabhängigen Signal des Betriebsparameters abgeleitetes Signal darstellt.

Die Bildung eines abgeleiteten Signals umfasst, dass ein zeitlicher Ausschnitt oder ein Zeitfenster aus dem zeitabhängigen Signal des Betriebsparameters ausgeschnitten wird, etwa durch eine Fensteroperation, insbesondere die Anwendung einer gewichteten Fensterfunktion. Die Bildung eines abgeleiteten Signals kann weiterhin umfassen, dass Merkmale aus dem zeitabhängigen Signal des Betriebsparameters extrahiert werden oder dass das zeitabhängige Signal des Betriebsparameters einer Filteroperation unterzogen wird. Die Filteroperation kann linear oder nicht-linear sein. Beispiele für eine lineare Filteroperation sind eine Fouriertransformation oder eine Wavelettransformation.

Der Betriebsparameter kann etwa eine Blutflussrate, eine Bluttemperatur, einen Bluthämatokrit in einem extrakorporalen Blutkreislauf, eine Dialysattemperatur bei einer Hämodialyse, eine Leitfähigkeit des Dialysats bei einer Hämodialyse, einen Blutdruck in einem extrakorporalen Blutkreislauf, insbesondere einen arteriellen Blutdruck, einen venösen Blutdruck oder eine Kombination eines arteriellen und eines venösen Blutdrucks, oder die Leistungsaufnahme einer Aktors, insbesondere einer Blutpumpe umfassen. Ein Betriebsparameterüberwachungssignal im Sinne der vorliegenden Offenbarung kann durch Kombination von mehreren Betriebsparameterüberwachungssignalen gebildet werden.

Das Computersystem umfasst weiterhin eine zweite Schnittstelle, die angepasst ist, ein Anwenderreaktionssignal zu empfangen, wobei ein Anwenderreaktionssignal ein Signal einer Anwendereingabe im Hinblick auf den Betriebszustand oder den Behandlungsverlauf oder die Abweichung von einem idealen oder komplikationsfreien Behandlungsverlauf oder Betriebszustand einer mit der Blutbehandlungsmaschine durchgeführten Blutbehandlung darstellt. Die erste und zweite Schnittstelle können als eine Schnittstelle ausgeführt sein

Der Betriebszustand oder Behandlungsverlauf oder die Abweichung von einem idealen oder komplikationsfreien Betriebszustand oder Behandlungsverlauf können sich auf den Patienten, auf die Blutbehandlungsmaschine oder auf das System aus Patient und Blutbehandlungsmaschine beziehen. Beispiele sind eine Diskonnektion der venösen Nadel von dem Zugang des Patienten, ein Zusetzten der Dialysatormembran (Clotting), eine Rezirkulation des behandelten Blutes in dem arteriellen Teil des extrakorporalen Blutkreislaufs oder eine Blutdruckkrise.

Das Anwenderreaktionssignal ist eines aus der Gruppe eines Anpassens einer Blutflussrate, oder der Ultrafiltrationsrate, eine Einleitung der Gabe eines Elektrolyts,eines manuell ausgelöster Alarms oder eines manuell ausgelöster Behandlungsstopps sein, eines Feststellens einer Blutdruckkrise, oder eines Feststellens einer Nadeldislokation.

Die Anwenderreaktion kann während einer laufenden Behandlung oder aufgrund einer Auswertung historischer Behandlungsdaten, etwa eines historischen Betriebsparameterüberwachungssignals erfolgen. Die Anwenderreaktion kann durch einen Patienten, durch einen Behandler oder durch einen Experten erfolgen.

Das Anwenderreaktionssignal kann mit einem Zeitstempel versehen sein, der den Zeitpunkt der Anwenderreaktion oder einer Anwendereingabe in Bezug auf den Behandlungsverlauf angibt,

Die Anwendereingabe liegt in dem zeitlichen Ausschnitt oder dem Zeitfenster oder benachbart dazu.

Das Computersystem umfasst vorteilhaft eine Speichereinheit, welche konfiguriert ist, eine Mehrzahl von Anwenderreaktionssignalen und Betriebsparameterüberwachungssignalen in Bezug zueinander zu speichern.

Das Computersystem umfasst weiterhin eine Auswerteeinheit, die angepasst ist, um Zuordnungs- oder Referenzdaten zu erzeugen, die eine Korrelation zwischen einer bestimmten Anwenderreaktion mit einem oder mehreren korrespondierenden Betriebsparameterüberwachungssignalen herstellen.

Die Zuordnungs- und Referenzdaten können in Form einer Funktion vorliegen, die eine Abbildung eines Betriebsparameterüberwachungssignals auf eine entsprechende Anwenderreaktion herstellt.

Das korrespondierende Betriebsparamterüberwachungssignal kann ein typisches Betriebsparameterüberwachungssignal, das zu der bestimmten Anwendereaktion passt, darstellen. Das zugeordnete Betriebsparamterüberwachungssignal kann eine Vielzahl von Betriebsparamterüberwachungssignalen, die zu einer bestimmten Anwenderreaktion passen oder eine Mittelung aus einer Vielzahl von Betriebsparamterüberwachungssignalen, die zu einer bestimmten Anwenderreaktion passen, darstellen. Die Mittelung kann eine lineare oder eine nicht-lineare Mittelung sein.

Das Erzeugen der Zuordnungs- und Referenzdaten kann unter Berücksichtigung eines maschinenspezifischen Parameters der Blutbehandlungsmaschine erfolgen, etwa in dem Sinne, dass eine Normierung unter Berücksichtigung des maschinenspezifischen Parameters stattfindet.

Das Erzeugen der Zuordnungs- oder Referenzdaten kann auf der Anwendung eines Lernalgorithmus beruhen, etwa auf der Anwendung eines neuronalen Netzes, bei der die Zuordnung zwischen einer bestimmten Anwenderreaktion und einem zugeordneten Betriebsparameterüberwachungssignal aus einer Vielzahl von Datentupeln gelernt wird. Ein Datentupel umfasst dabei jeweils eine bestimmte Anwenderreaktion und ein entsprechendes Betriebsparameterüberwachungssignal, das zur Zeit der Anwendereaktion aufgenommen wurde.

Die Auswerteeinheit ist weiterhin angepasst, ein bestimmtes Betriebsparameterüberwachungssignal, vorzugsweise ein momentan aufgenommenes Betriebsparameterüberwachungssignal einer laufenden Blutbehandlung, mit den Zuordnungs- oder Referenzdaten abzugleichen, um daraus einen Betriebszustand oder Behandlungsverlauf, oder eine Abweichung von einem idealen oder komplikationsfreien Betriebszustand oder Behandlungsverlauf der Blutbehandlungsmaschine abzuleiten, insbesondere einen momentanen Betriebszustand oder Behandlungsverlauf, oder eine momentane Abweichung von einem idealen oder komplikationsfreien Betriebszustand der Blutbehandlungsmaschine oder dem Behandlungsverlauf der laufenden Blutbehandlung, oder um eine typische Anwenderreaktion hierauf abzuleiten.

Das bestimmte Betriebsparameterüberwachungssignal und die entsprechende typische Anwenderreaktion können in der Art eines iterativen Lernprozesses zur Erzeugung weiterer und/oder verbesserter Zuordnungs- und Referenzdaten verwendet werden.

Der momentane Betriebszustand oder die momentane Abweichung von einem idealen oder komplikationsfreien Betriebszustand kann mit einer typischen oder adäquaten Anwenderreaktion hierauf verknüpft sein, etwa wenn der momentane Betriebszustand ein Alarmzustand und die adäquate Nutzerreaktion eine Unterbrechung der Blutbehandlung ist.

Die Blutbehandlungsmaschine kann so konfiguriert sein, dass über eine Benutzerschnittstelle ein Vorschlag an den Anwender ausgegeben wird, die typische oder adäquate Anwenderreaktion vorzunehmen.

Die Blutbehandlungsmaschine kann weiterhin so konfiguriert sein, dass eine der typischen oder adäquaten Benutzerreaktion entsprechende Anpassung der Betriebsparameter, etwa eine Reduktion der Blutflussrate oder eine Behandlungsunterbrechung automatisch vorgenommen werden.

Eine Ausführungsform betrifft computerimplementiertes Verfahren zur Erkennung eines Betriebszustandes einer Blutbehandlungsmaschine oder eines Behandlungsverlaufs einer mit der Blutbehandlungsmaschine durchgeführten Blutbehandlung oder einer Abweichung von einem idealen oder komplikationsfreien Betriebszustand oder Behandlungsverlauf. Das computerimplementierte Verfahren umfasst folgende Schritte:
Empfangen eines Betriebsparameterüberwachungssignals, wobei das Betriebsparameterüberwachungssignal ein zeitabhängiges Signal eines Sensors zur Überwachung eines Betriebsparameters der Blutbehandlungsmaschine oder ein von dem zeitabhängigen Signal des Betriebsparameters abgeleitetes Signal darstellt,
Empfangen eines Anwenderreaktionssignals, wobei das Anwenderreaktionssignal ein Signal einer Anwendereingabe im Hinblick auf den Betriebszustand oder den Behandlungsverlauf oder die Abweichung von einem idealen oder komplikationsfreien Betriebszustand oder Behandlungsverlauf darstellt, abspeichern einer Mehrzahl von Anwenderreaktionssignalen und Betriebsparameterüberwachungssignalen in Bezug zueinander,
Erzeugen von Zuordnungs- oder Referenzdaten, die eine Zuordnung oder Korrelation einer bestimmten Anwenderreaktion mit einem zugeordneten Betriebsparameterüberwachungssignal herstellen,
Speichern der Zuordnungs- oder Referenzdaten,
Empfangen eines bestimmten Betriebsparameterüberwachungssignals zu einem bestimmten Zeitpunkt während der Blutbehandlung, und
Vergleichen des bestimmten Betriebsparameterüberwachungssignals mit den Zuordnungs- oder Referenzdaten, um daraus einen momentanen Betriebszustand oder Behandlungsverlauf, oder eine momentane Abweichung von einem idealen oder komplikationsfreien Betriebszustand oder Behandlungsverlauf zu dem bestimmten Zeitpunkt oder eine typische Anwenderreaktion hierauf abzuleiten.

Auf das computerimplementierte Verfahren können dieselben Modifikationen angewendet werden wie auf das beschriebene Computersystem.

### Kurze Beschreibung der Zeichnungen

Figur 1 zeigt ein Dialysesystem mit einer Hämodialysemaschine in einer Ausführungsform gemäß der vorliegenden Lehre.
Figur 2 zeigt ein Blutbehandlungssystem in einer Ausführungsform gemäß der vorliegenden Lehre.
Figur 3 zeigt ein Ablaufdiagram eines computerimplementierten Verfahrens in einer Ausführungsform gemäß der vorliegenden Lehre.
Figur 4 zeigt die Zuordnung zwischen Anwendereingabe und Signalmuster am Beispiel eines venösen und eines arteriellen Druckmusters.

### Detaillierte Beschreibung der Zeichnungen

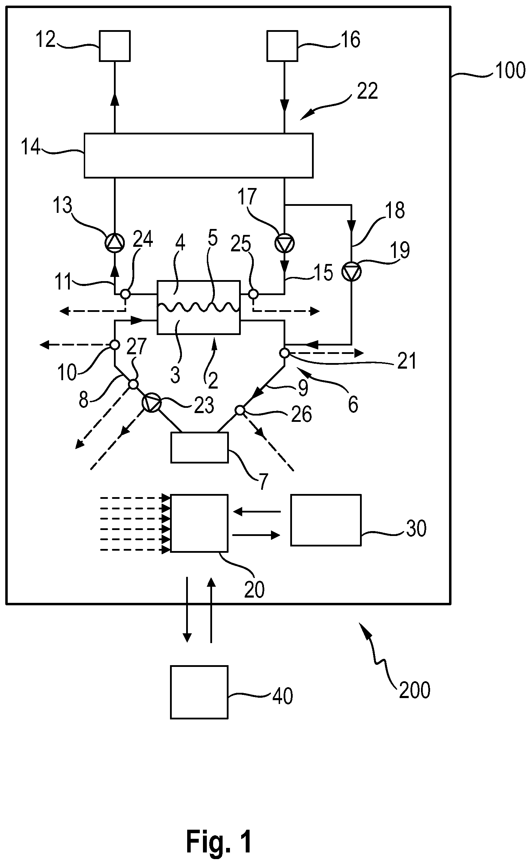

Figur 1 zeigt ein Dialysesystem mit 200 mit einer Dialysemaschine 100 und einer zentralen Überwachungseinheit 40.

Die Dialysemaschine 20 weist eine Blutbehandlungseinheit 2 (Dialysator) mit einer Dialysierflüssigkeitskammer 4 in einem Dialysierflüssigkeitskreislauf 22 auf, die durch eine semipermeable Membran 5 von einer Blutkammer 3 in einem Blutkreislauf getrennt ist.

Aufbereitete Dialysierflüssigkeit wird von einer Dialysierflüssigkeitsquelle 16 bereitgestellt und von einer Dialysierflüssigkeitspumpe 17 über die Dialysierflüssigkeitszuführleitung 15 in die Dialysierflüssigkeitskammer 4 befördert. Verbrauchte Dialysierflüssigkeit wird von der Dialysierflüssigkeitspumpe 13 über die Dialysierflüssigkeitsabführleitung 11 von der Dialysierflüssigkeitskammer 4 weg und einem Abfluss 12 zugeführt. In einer alternativen Ausführungsform, kann die verbrauchte Dialyierflüssigkeit auch aufbereitet werden.

In der Dialysierflüssigkeitskammer 4 nimmt die Dialysierflüssigkeit die harnpflichtigen Substanzen auf, die über die Dialysatormembran 5 von dem in der Blutkammer 3 befindlichen Blut in die Dialysierflüssigkeit übergehen.

Eine Bilanziereinheit 14 sorgt für die Bilanzierung zwischen frischer und verbrauchter Dialysierflüssigkeit. Zur Bilanzierung können Flussensoren, Waagen oder Bilanzkammern eingesetzt werden. Im letztgenannten Fall kann die Bereitstellung einer zweiten Dialysierflüssigkeitspumpe entbehrlich sein.

In der dargestellten optionalen Ausführung einer Hämodiafltrationsmaschine ist zusätzlich eine Substituatleitung 18 vorgesehen, über die mit einer Substituatpumpe 19 geförderte gefilterte Dialysierflüssigkeit als Substituat dem zu behandelnden Blut zugegeben wird. Der hierzu erforderliche Filter ist zur Vereinfachung der Darstellung nicht gezeigt. Die Zugabe der Substitutionsflüssigkeit kann wie gezeigt stromab des Dialysators erfolgen (Postdilution) oder stromauf des Dialysators (Prädilution). Eine Kombination dieser Verfahren ist möglich.

Das zu behandelnde Blut wird dem Patienten 7 über einen Patientenzugang entnommen und von der Blutpumpe 23 über die arterielle Blutleitung 8 des extrakorporalen Blutkreislaufs 6 der Blutkammer 3 des Dialysators 2 zugeführt, dort von unerwünschten Blutinhaltsstoffen gereinigt, und über die venöse Blutleitung 9 dem Patienten 7 zurückgegeben.

Zur Überwachung der Blutbehandlung weist die Dialysemaschine 100 verschiedene Sensoren auf. So kann im Dialysierflüssigkeitskreislauf ein Leitfähigkeitssensor 25 in der Dialyserflüssigkeitszuführleitung vorgesehen sein, um die Leitfähigkeit im Frischdialysat zu bestimmen. Ein Leitfähigkeitssensor 24 in der Dialysierflüssigkeitsabführleitung kann vorgesehen sein, um die Leitfähigkeit im verbrauchten Dialysat zu bestimmen. Das Signal des Leitfähigkeitssensors 24 und das Signal des Leitfähigkeitssensors 25 oder eine Kombination des Signals des Leitfähigkeitssensors 24 und 25 bilden jeweils ein Betriebsparameterüberwachungssignal.

Ein Betriebsparameterüberwachungssignal, das auf der Messung der Signale des Leitfähigkeitssensors 24 und 25 in Kombination beruht, ist die Dialysance. Den Hintergrund dazu liefert die Überwachung der Blutreinigungsleistung oder Clearance, die bei der Überwachung der extrakorporalen Blutbehandlung von Bedeutung ist. Die Clearance K ist definiert, als der Anteil des Blutflusses durch die Blutkammer, die vollständig von Toxinen, insbesondere Urea, gereinigt wird. In der Praxis wird an Stelle der Clearance die Dialysance bestimmt, bei der die Durchgängigkeit der Filtermembran für in der Dialysierflüssigkeit enthaltenen Elektrolyte gemessen wird. Dazu wird die Konzentration eines oder mehrerer Elektrolyte in der Dialysierflüssigkeit im Dialysierflüssigkeitskreislauf stromauf der Dialysierflüssigkeitskammer 4 mit Hilfe eines Boluserzeugungsmittels (nicht gezeigt) verändert und die daraus resultierende Änderung der Konzentration stromab der Dialysierflüssigkeitskammer bestimmt. Die geänderte Konzentration der einen oder mehreren Elektrolyte stromauf sowie stromab der Dialysierflüssigkeitskammer beeinflusst die Leitfähigkeit der Dialysierflüssigkeit und wird mit den Leitfähigkeitssensoren 25 stromauf und 24 stromab der Dialysierflüssigkeitskammer 4 gemessen. Ein solches Verfahren zur Bestimmung der Clearance während der laufenden Behandlung wird als online Clearance Monitoring bezeichnet und ist in dem europäischen Patent EP 0 911 043 der Anmelderin offenbart, deren Offenbarung vollumfänglich in die vorliegende Anmeldung einbezogen wird. Ein kommerzielles Verfahren, das auf diesem Prinzip beruht, wird von der Firma Fresenius Medical Care Deutschland GmbH unter der Bezeichnung OCM (Online Clearance Monitor) vertrieben.

Als Sensoren im extrakorporalen Blutkreislauf 6 können in der arteriellen Blutleitung 8 ein arterieller Drucksensor 27 und ein arterieller Temperatursensor 10 und in der venösen Blutleitung 9 ein venöser Drucksensor 26 sowie ein venöser Temperatursensor 21, vorgesehen sein. Von der Blutpumpe 23 wird ein Leistungsaufnahmesignal bereitgestellt. Das Signal des venösen Drucksensors 26, das Signal des arteriellen Drucksensors 27, das Signal des venösen Temperatursensors 21, das Signal des arteriellen Temperatursensors 10, sowie das Leistungsaufnahmesignal der Blutpumpe 23 sind jeweils zeitabhängig und bilden jeweils ein Betriebsparamterüberwachungssignal. Zusätzlich kann in der arteriellen und/oder der venösen Blutleitung ein Hämatokritsensor (nicht dargestellt) vorgesehen sein, der jeweils ein Betriebsparameterüberwachungssignal bereitstellt.

Betriebsparameterüberwachungssignale werden während einer laufenden Behandlung an die zentrale Verarbeitungseinheit 20 übertragen und dort aufgezeichnet und nach Beendigung der Behandlung an die zentrale Überwachungseinheit 40 übertragen. Alternativ werden Betriebsparameterüberwachungssignale kontinuierlich während der laufenden Behandlung an die zentrale Überwachungseinheit 40 übertragen. Die zentrale Überwachungseinheit 40 kann eine Datenbank umfassen.

Die Dialysemaschine 100 weist weiterhin eine Eingabeeinheit 30 auf, mit deren Hilfe eine Anwendereingabe vornehmen werden kann betreffend die Blutbehandlung oder deren Ablauf, oder einer Abweichung von einem idealen oder komplikationsfreien Behandlungsverlauf oder Betrieb der Dialysemaschine. Die Anwenderreaktion kann eine übliche Anwendereingabe im Rahmen der laufenden Blutbehandlung, die Auslösung eines Alarms oder Behandlungsstopps oder die Angabe der Ursache eines Alarms betreffen. Die Anwendereingabe wird an die zentrale Verarbeitungseinheit 20 weitergegeben und dort weiterverarbeitet. Während der laufenden Behandlung oder nach deren Beendigung wird ein die Anwendereingabe enthaltendes Signal an die zentrale Überwachungseinheit übertragen.

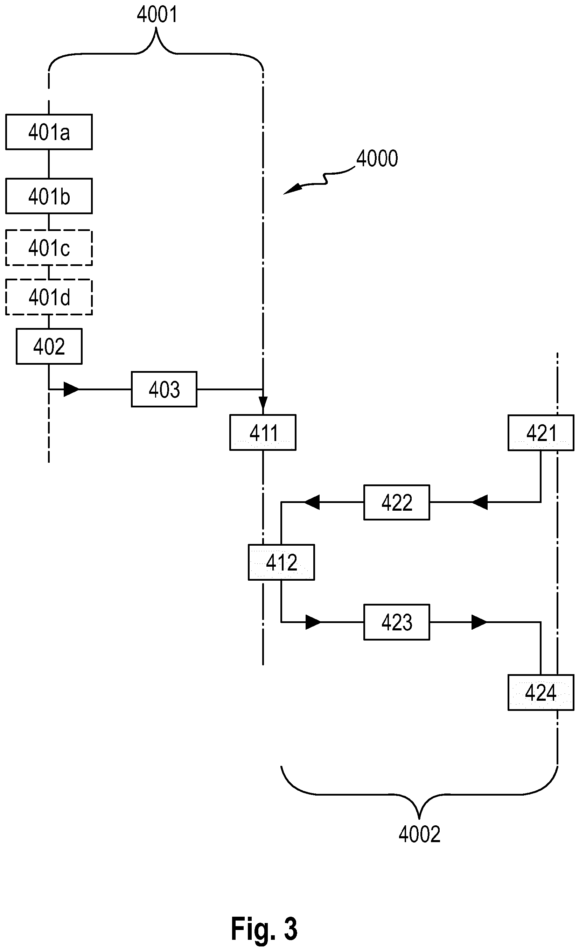

In der zentralen Überwachungseinheit werden die Anwendereingabe und das entsprechende zugeordnete Betriebsparameterüberwachungssignal gespeichert und entsprechend der im Zusammenhang mit Figur 3 beschriebenen Datenakquisitions- oder Lernphase 4001 und/oder der Überwachungsphase 4002 verwendet.

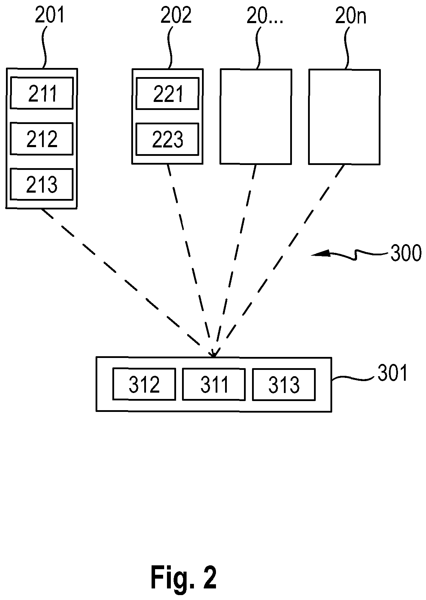

Figur 2 zeigt schematisch ein Blutbehandlungssystem gemäß der vorliegenden Lehre.

Das Blutbehandlungssystem 300 weist eine Vielzahl von Blutbehandlungsmaschinen 201, ..., 20n auf, deren Betriebsparameter laufend überwacht werden.

Die Blutbehandlungsmaschinen 201, ..., 20n verfügen jeweils über mindestens einen Sensor 211 zur Überwachung eines Betriebsparameters der Blutbehandlung und zur Erzeugung eines zeitabhängigen Sensorsignals oder eines von diesem abgeleiteten Signals.

Weiterhin verfügen die Blutbehandlungsmaschinen 201, ..., 20n jeweils über mindestens eine Ein-/Ausgabeeinheit 212, die angepasst eine Anwendereingabe im Hinblick auf den Betriebszustand oder den Behandlungsverlauf oder die Abweichung von einem idealen oder komplikationsfreien Behandlungsverlauf oder Betriebszustand einer mit der Blutbehandlungsmaschine durchgeführten Blutbehandlung vorzunehmen.

Blutbehandlungsmaschinen 201, ..., 20n verfügen weiterhin jeweils über eine Schnittstelle 213 zur Kommunikation mit der Zentraleinheit 301 und zur Übertragung von Anwendereingaben und Sensorsignalen an die Zentraleinheit 301.

Die Zentraleinheit 301 verfügt über eine Schnittstelle 312 zur Kommunikation mit den Blutbehandlungsmaschinen 201, ..., 20n und zum Empfangen von Anwendereingaben und Sensorsignalen von den Blutbehandlungsmaschinen.

Die Zentraleinheit 301 kann als Einzelsystem oder als verteiltes System implementiert sein. Die Zentraleinheit kann Teil einer Client-/Server Architektur sein und/oder als Teil eines Cloudnetzwerks ausgeführt sein. Die Zentraleinheit 301 verfügt über eine Speichereinheit 311, welche konfiguriert ist, eine Mehrzahl von Anwenderreaktionssignalen und Betriebsparameterüberwachungssignalen in Bezug zu einander zu speichern, und über eine Auswerteeinheit 313, die angepasst ist, um Zuordnungs- oder Referenzdaten zu erzeugen, die eine Korrelation einer bestimmten Anwenderreaktion mit einem zugeordneten Betriebsparameterüberwachungssignal herstellen. Die Speichereinheit ist weiterhin angepasst, die Zuordnungs- und Referenzdaten zu speichern. Die Speichereinheit 311 kann als Datenbank ausgeführt sein. Die Auswerteeinheit 313 hat Zugriff auf die in der Speichereinheit 311 gespeicherten Zuordnungs- und Referenzdaten.

Die Auswerteeinheit 313 ist weiterhin angepasst, ein bestimmtes Betriebsparameterüberwachungssignal, das zu einem bestimmten Zeitpunkt von einer der Blutbehandlungsmaschinen 201, ..., 20n während der Blutbehandlung aufgenommen wurde von einer der Blutbehandlungsmaschinen, hier der Blutbehandlungsmaschine 202, zu empfangen und mit den Zuordnungs- oder Referenzdaten abzugleichen, um daraus einen Betriebszustand, einen Behandlungsverlauf, oder eine Abweichung von einem idealen oder komplikationsfreien Betriebszustand oder Behandlungsverlauf zu dem bestimmten Zeitpunkt oder eine geeignete Anwenderreaktion darauf abzuleiten.

Die Zentraleineinheit 301 ist angepasst an einen Betriebszustand, den Behandlungsverlauf, oder die Abweichung von einem idealen oder komplikationsfreien Betriebszustand oder Behandlungsverlauf zu dem bestimmten Zeitpunkt oder die geeignete Anwenderreaktion an die entsprechende der Blutbehandlungsmaschinen 201, ... , 20n, hier an die Blutbehandlungsmaschine 202, zurückzugeben.

Die Blutbehandlungsmaschine 202 ist angepasst, geeignet auf den Betriebszustand, den Behandlungsverlauf, oder die Abweichung von einem idealen oder komplikationsfreien Betriebszustand oder Behandlungsverlauf zu dem bestimmten Zeitpunkt die geeignete Anwenderreaktion zu reagieren.

Dazu verfügt die Blutbehandlungsmaschine 202 über eine Ausgabeeinheit 221 zur Ausgabe eines Alarms als Antwort auf die abgeleitete Abweichung von einem idealen oder komplikationsfreien Betriebszustand oder Behandlungsverlauf der Blutbehandlungsmaschine oder die typische Anwenderreaktion.

Weiterhin verfügt die Blutbehandlungsmaschine 202 über eine Steuereinheit 223 zum Ansteuern der Blutbehandlungsmaschine als Antwort auf die abgeleitete Abweichung von einem idealen oder komplikationsfreien Betriebszustand oder Behandlungsverlauf der Blutbehandlungsmaschine oder die typische Anwenderreaktion. In einer Ausführungsform ist die Steuereinheit angepasst, als Antwort auf die Abweichung eine Unterbrechung der Blutbehandlung einzuleiten.

Figur 3 ist ein Ablaufdiagram eines offenbarungsgemäßen computerimplementierten Verfahrens 4000 zur Bestimmung eines Betriebszustands einer Blutbehandlungsmaschine, eines Behandlungsverlaufs einer Blutbehandlung oder der Abweichung von einem idealen oder komplikationsfreien Betriebszustand oder Behandlungsverlauf bei einer Blutbehandlung oder einer geeigneten Anwenderreaktion hierauf.

Die einzelnen Schritte des computerimplementierten Verfahrens können von den Komponenten des Dialysesystems 200 (vergleiche Fig. 1) oder von den Komponenten des Blutbehandlungssystems 300 (vergleiche Fig. 2) ausgeführt werden.

Das computerimplementierte Verfahren 4000 umfasst eine Datenakquisitions- oder Lernphase 4001 sowie eine Überwachungsphase 4002.

Während der Durchführung einer Blutbehandlung mit einer Blutbehandlungsmaschine 201, ... 20n, insbesondere einer Dialysebehandlung mit einer Dialysemaschine 100, wird mindestens ein Betriebsparameter der Blutbehandlungsmaschine kontinuierlich überwacht 401a und sein zeitabhängiger Verlauf eines Messignals des überwachten Betriebsparameters wird als Betriebsparameterüberwachungssignal 401b gespeichert. Das zeitabhängige Messsignal des überwachten Betriebsparameters kann ein zeitabhängiges Signal eines Sensors der Blutbehandlungsmaschine, etwa ein Leitfähigkeitssignal des Frischdialysats, ein Leitfähigkeitssignal des verbrauchten Dialysats, ein Temperatursignal der arteriellen oder venösen Bluttemperatur, ein Drucksignal des arteriellen oder venösen Temperatursensors oder ein Leistungsaufnahmesignal der Blutpumpe oder ein anderes zeitabhängiges Messsignal eines Betriebsparameters sein.

Im Hinblick auf den Behandlungsverlauf, oder auf eine Abweichung von einem idealen oder komplikationsfreien Behandlungsverlauf oder Betriebszustand während der Blutbehandlung erfolgt eine Anwendereingabe 402.

Die Anwendereingabe 402 kann eine Reaktion des Anwenders auf eine festgestellte Situation im Rahmen einer laufenden Blutbehandlung darstellen, etwa die Reduzierung der Blutflussrate, in Folge eines zunehmenden arteriellen Drucks, den der Anwender festgestellt hat. Andere Beispiele sind die Reduzierung der Ultrafiltrationsrate oder die Einleitung der Gabe eines Elektrolyts wegen einer festgestellten Blutdruckkrise.

Die Anwendereingabe 402 kann einen manuell ausgelösten Alarm oder einen manuell ausgelösten Behandlungsstopp umfassen.

Alternativ oder zusätzlich kann ein automatisch generierter Alarm oder ein manuell ausgelöster Alarm mit einer Aufforderung an den Anwender verbunden sein, eine Ursache für den Alarm anzugeben. So kann dem Anwender vorteilhaft eine Auswahlmöglichkeit unter verschiedenen möglichen Ursachen für den aufgetretenen Alarm angeboten werden, etwa in Form eines Drop-down Menüs. Die Anwendereingabe 402 umfasst in diesem Fall die Angabe der Ursache für den Alarm. Die Anwendereingabe kann durch einen Behandler oder durch den behandelten Patienten erfolgen. In einer Ausführungsform wird die Anwendereingabe durch einen Zeitstempel ergänzt, der den Zeitpunkt der Anwendereingabe in Bezug auf den Behandlungsverlauf angibt, etwa die Zeit, die seit Beginn der Blutbehandlung vergangen ist.

Nach dem Erreichen des Behandlungsendes werden das aufgezeichnete Sensorsignal zusammen mit einer oder mehreren während der Blutbehandlung vorgenommenen Anwendereingaben von der Blutbehandlungsmaschine an eine zentrale Überwachungseinheit übertragen 403. Vor dem Übertragen an die zentrale Recheneinheit kann das Sensorsignal einer Signalverarbeitung 401c und/oder einer Merkmalsextraktion 401d unterzogen werden. Die Signalverarbeitung kann eine Trendanalyse des Sensorsignals umfassen, etwa die Analyse eines Langzeittrends. In einer Ausführungsform umfasst die Signalverarbeitung 401c eine Fensteroperation im Bereich des Zeitpunkts der Benutzereingaben, um einen Signalausschnitt zu erzeugen (Windowing), oder die Anwendung eines linearen oder nicht-linearen Filters umfassen. Beispiele für eine lineare Operation sind eine Fouriertransformation des Signalausschnitts, oder eine Waveletttransformation. In einer alternativen Ausführungsform können Signalverarbeitung 401c und/oder Merkmalsextraktion 401d auf der Seite der zentralen Überwachungseinheit erfolgen. In einer weiteren alternativen Ausführungsform erfolgt die Übertragung 403 des Senorsignals an den zentralen Überwachungseinheit kontinuierlich während der laufenden Blutbehandlung.

In der zentralen Auswerteeinheit erfolgt eine Erzeugung 411 von Zuordnungs- und Referenzdaten, die eine Zuordnung oder Korrelation zwischen jeweils einer bestimmten Anwendereingabe und dem entsprechenden Sensorsignal herstellen.

Vorteilhaft wird dabei die Abfolge der Schritte 401a bis 411 als Datenakquistions- oder Lernphase 4001 zur Erzeugung der Zuordnungs- und Referenzdaten für eine Vielzahl von Behandlungen, vorzugsweise unter Verwendung einer Vielzahl von Blutbehandlungsmaschinen 201, ..., 20n durchlaufen. Bei dem Schritt der Erzeugung 411 der Zuordnungs- und Referenzdaten kann die Zuordnung von zwischen Anwendereingabe und Sensorsignal in der Form einer Lernphase eines Lernalgorithmus, vorzugsweise eines neuronalen Netzes erfolgen. Vereinfacht ausgedrückt stellen die Zuordnungs- oder Referenzdaten eine Zuordnung zwischen einer bestimmten Anwendereingabe und einem dieser Anwendereingabe entsprechenden typischen Signalverlauf eines bestimmten Betriebsparameters (Signalmuster) her. Z.B. es stellt sich heraus, dass bei einer Dislokation der venösen Nadel der Signalverlauf des arteriellen Blutdrucks und der Signalverlauf des venösen Blutdrucks ein charakteristisches Muster aufweisen. In diesem Fall stellen die Zuordnungs- und Referenzdaten eine Zuordnung zwischen diesem charakteristischen Signalmuster und der von dem Anwender festgestellten Abweichung von dem komplikationsfreien Behandlungsverlauf in Form einer Anwendereingabe her. Die Zuordnung kann so erfolgen, dass dem Behandlungsverlauf eine bestimmte Ursache zugeordnet wird, oder eine adäquate Anwenderreaktion hierauf, also im Falle der Dislokation der venösen Nadel, die Einleitung eines sofortigen Behandlungsstopps.

In einer Ausführungsform umfassen die Zuordnungs- oder Referenzdaten zu einer bestimmten Anwendereingabe, eine Vielzahl von zugeordneten Signalmustern, zusammen mit der Angabe einer Häufigkeit, mit der ein bestimmtes Signalmuster auftritt, oder eine Wahrscheinlichkeitsverteilung über verschiedene zugeordnete Signalmuster.

In einer alternativen Ausführungsform umfassen die Zuordnungs- und Referenzdaten zu einem bestimmten Signalmuster eine Vielzahl von Anwenderreaktionen, vorzugsweise verbunden mit einer Wahrscheinlichkeitsverteilung über die Anwenderreaktionen, wobei die Anwenderreaktion, für die die größte Wahrscheinlichkeit angegeben wurde, vorzugsweise als die adäquateste Anwenderreaktion gekennzeichnet ist.

In einer alternativen Ausgestaltung erfolgt die Anwendereingabe nicht oder nicht ausschließlich online während der Blutbehandlung, sondern es werden die aufgezeichneten Sensordaten offline durch einen Experten im Hinblick darauf untersucht, eine Abweichung von einem idealen oder komplikationsfreien Betriebszustand oder Behandlungsverlauf zu erkennen, und/oder eine Ursache für diese Abweichung oder eine adäquate Reaktion hierauf anzugeben. Diese Angabe der Ursache für die Abweichung oder die Reaktion darauf stellt in dieser Ausführungsform die Anwendereingabe dar, die wiederum mit dem Sensorsignal zugeordnet und/oder mit diesem korreliert wird, um Zuordnungs- oder Referenzdaten zu erzeugen, wie oben beschrieben. In einer Ausführungsform kann bei der Erzeugung der Zuordnungs- und Referenzdaten einer Anwendereingabe durch einen Experten ein unterschiedlich starkes Gewicht gegeben werden, in Abhängigkeit von einer Erfahrungsstufe des Experten.

Die Zuordnungs- und Referenzdaten können in einer nachfolgenden Überwachungsphase 4002 verwendet werden, um eine laufende Blutbehandlung zu überwachen. Für die zu überwachende Blutbehandlung werden Sensordaten eines Betriebsparameters kontinuierlich aufgenommen 421 und an die zentrale Überwachungseinheit 40, 301 übertragen 422 und dort empfangen. In der zentralen Überwachungseinheit erfolgt ein Vergleich 412 der übertragenen Sensordaten oder eines Ausschnitts daraus mit den Zuordnungs- und Referenzdaten. Wird eine Ähnlichkeit oder eine Korrelation des Musters des übertragenen Sensorsignals oder eines Ausschnitts daraus mit einem typischen Signalmuster, das in den Zuordnung- oder Referenzdaten enthalten ist, festgestellt, so wird davon die zugeordnete Anwendereingabe abgeleitet. In einer Ausführungsform wird eine Korrelation zwischen einem übertragenen Sensorsignal und einem typischen Signalmuster in der Art festgestellt, dass mit dem typischen Signalmuster eine höchste Korrelation verglichen mit anderen in der zentralen Überwachungseinheit abgelegten Signalmustern besteht.

Auf diese Weise kann eine Abweichung von einem idealen oder komplikationsfreien Behandlungsverlauf festgestellt werden, und/oder eine Ursache hierfür oder eine adäquate Anwenderreaktion hierauf festgestellt werden. Die festgestellte Anwendereingabe oder eine davon abgeleitete Abweichung von einem idealen oder komplikationsfreien Betriebszustand oder Behandlungsverlauf der Blutbehandlungsmaschine wird an die Blutbehandlungsmaschine übertragen 423. Die Blutbehandlungsmaschine kann auf die übertragene Anwendereingabe oder den abgeleitete Abweichung von dem idealen oder komplikationsfreien Betriebszustand oder Behandlungsverlauf geeignet reagieren 424. So kann die Blutbehandlungsmaschine, einen Alarm ausgeben, einen Behandlungsstopp oder eine Anpassung von Behandlungsparametern durchführen, entsprechend der festgestellten Anwendereingabe.

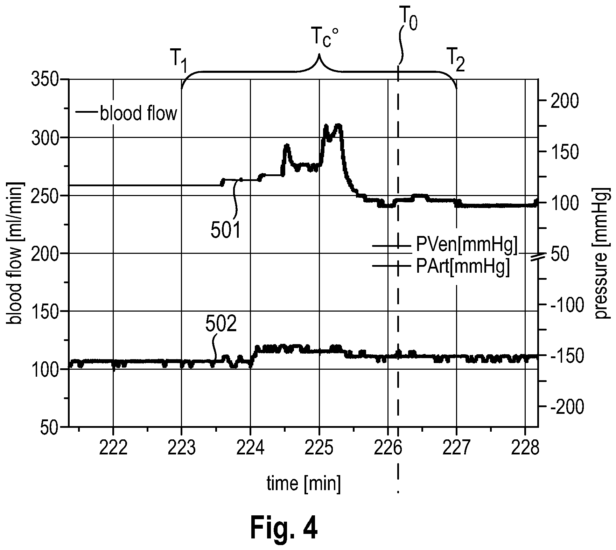

Figur 4 zeigt die Zuordnung zwischen einer Anwendereingabe und einem Signalmuster am Beispiel eines venösen und eines arteriellen Druckmusters zum Zeitpunkt einer venösen Nadeldislokation bei einer Blutbehandlung mit einer Blutbehandlungsmaschine 100, wie sie im Zusammenhang mit der Figur 4 beschrieben ist.

Der zeitabhängige Verlauf des Signals des venösen Drucksensors 26 ist mit 501, der zeitabhängige Verlauf des Signals des arteriellen Drucksensors 27 ist mit 502 gekennzeichnet. Zwischen dem Zeitpunkt T1 und dem Zeitpunkt T2 kommt es zu einer Dislokation der venösen Nadel. Zum Zeitpunkt T0 erfolgt eine Anwendereingabe, etwa die Einleitung eines Behandlungsstopps und/oder die Auslösung eines Alarms, vorteilhaft verbunden mit der Angabe einer entsprechenden Ursache für den Alarm.

Entsprechend der Anwendereingabe zum Zeitpunkt T0 wird zwischen den Zeitpunkten T1 und T2 jeweils ein Ausschnitt aus dem venösen Drucksignal und aus dem arteriellen Drucksignal gebildet (windowing). Die Signalausschnitte des venösen Drucksignals und des arteriellen Drucksignals bilden zusammen mit der Anwendereingabe ein Zuordnungs- oder Referenzdatum im Sinne der vorliegenden Offenbarung.

## Patentansprüche

1. Blutbehandlungssystem (300) mit mindestens einer Blutbehandlungsmaschine (100, 201, 202, 20.., 20n) zur Durchführung einer Blutbehandlung mit mindestens einem Sensor (211, 10, 21, 24, 25, 20, 27) zur Bestimmung eines zeitabhängigen Verlaufs eines Betriebsparameters während der Blutbehandlung, und mit einem Computersystem zur Erkennung eines Betriebszustandes einer der Blutbehandlungsmaschinen (100, 201, 202, 20.., 20n) oder eines Behandlungsverlaufs einer Blutbehandlung oder einer Abweichung von einem idealen oder komplikationsfreien Betriebszustand oder Behandlungsverlauf, wobei das Computersystem umfasst:
eine erste Schnittstelle (20, 312), die angepasst ist, ein Betriebsparameterüberwachungssignal zu empfangen, wobei das Betriebsparameterüberwachungssignal ein von einem zeitabhängigen Signal abgeleitetes Signal des Sensors zur Überwachung eines Betriebsparameters der den Sensor aufweisenden Blutbehandlungsmaschine (100, 201, 202, 20.., 20n) darstellt, wobei eine Bildung des abgeleiteten Signals umfasst, dass ein zeitlicher Ausschnitt oder ein Zeitfenster aus dem zeitabhängigen Signal des Betriebsparameters ausgeschnitten wird,
eine zweite Schnittstelle (20, 312), die angepasst ist, ein Anwenderreaktionssignal zu empfangen, wobei ein Anwenderreaktionssignal ein Signal einer Anwendereingabe im Hinblick auf den Betriebszustand oder den Behandlungsverlauf oder die Abweichung von einem idealen oder komplikationsfreien Behandlungsverlauf oder Betriebszustand einer mit der den Sensor aufweisenden der Blutbehandlungsmaschinen (100, 201, 202, 20.., 20n) durchgeführten Blutbehandlung darstellt, wobei die Anwendereingabe in dem Ausschnitt oder dem Zeitfenster oder einem benachbarten Ausschnitt oder Zeitfenster des Ausschnitts oder des Zeitfensters liegt, und wobei das Anwenderreaktionssignal ein Anpassen einer Blutflussrate oder einer Ultrafiltrationsrate, eine Einleitung der Gabe eines Elektrolyts, ein Feststellen einer Blutdruckkrise, oder ein Feststellen einer Nadeldislokation, oder eine Auslösung eines Alarms oder eines Behandlungsstopps ist,
eine Auswerteeinheit (40, 313), die angepasst ist, um Zuordnungs- oder Referenzdaten zu erzeugen, die eine Korrelation eines bestimmten Anwenderreaktionssignals mit einem zugeordneten Betriebsparameterüberwachungssignal herstellen,
eine Speichereinheit (311), welche konfiguriert ist, die Zuordnungs- und Referenzdaten abzuspeichern,
wobei die Auswerteeinheit (40, 313) weiterhin angepasst ist, ein bestimmtes Betriebsparameterüberwachungssignal mit den Zuordnungs- oder Referenzdaten abzugleichen, um daraus eine typische Anwenderreaktion hierauf abzuleiten,
wobei eine der Blutbehandlungsmaschinen (100, 201, 202, 20.., 20n) eine Steuereinheit (223) aufweist zum Einleiten einer Unterbrechung der Blutbehandlung als Antwort auf eine Abweichung einer typischen Anwenderreaktion an der die Steuereinheit aufweisenden Blutbehandlungsmaschine von der abgeleiteten typischen Anwenderreaktion, und/oder
wobei eine der Blutbehandlungsmaschinen (100, 201, 202, 20.., 20n) konfiguriert ist, über eine Benutzerschnittstelle einen Vorschlag an den Anwender auszugeben,
die abgeleitete typische Anwenderreaktion vorzunehmen, und/oder wobei eine der Blutbehandlungsmaschinen (100, 201, 202, 20.., 20n) konfiguriert ist, eine der abgeleiteten typischen Anwenderreaktion
entsprechende Anpassung eines Betriebsparameter automatisch vorzunehmen, und/oder
wobei das Blutbehandlungssystem (300) eine Ausgabeeinheit (212) zur Ausgabe eines Alarms als Antwort auf eine Abweichung einer typischen Anwenderreaktion an einer der Blutbehandlungsmaschine (100, 201, 202, 20.., 20n) von der abgeleiteten typischen Anwenderreaktion aufweist.

2. Blutbehandlungssystem (300) nach Anspruch 1, wobei die Auswerteeinheit (40, 313) angepasst ist, beim Erzeugen der Zuordnungs- oder Referenzdaten einen Lernalgorithmus anzuwenden, insbesondere ein neuronales Netz.

3. Blutbehandlungssystem (300) nach Anspruch 1, wobei die Blutbehandlungsmaschine (100, 201, 202, 20.., 20n) eine Dialysemaschine (100) ist, und wobei der Sensor (211, 10, 21, 24, 25, 20, 27) ausgewählt ist aus einem Bluttemperatursensor (10, 21), einem Hämatokritsensor, einem Blutdrucksensor (20, 27), einem Leitfähigkeitssensor (24, 25) zur Bestimmung einer Leitfähigkeit eines Dialysats oder einem Sensor zur Bestimmung der Leistungsaufnahme eines Aktors (23), vorzugsweise einer Blutpumpe (23).

4. Blutbehandlungssystem (300) nach einem der Ansprüche 1 bis 3, wobei eine Auswerteeinheit (20) der Blutbehandlungsmaschine (100, 201, 202, 20.., 20n) angepasst ist, das Anwendereingabesignal mit einem Zeitstempel zu versehen, der den Zeitpunkt der Anwendereingabe in Bezug auf den Behandlungsverlauf angibt, und wobei die Auswerteeinheit des Computersystems oder die Auswerteeinheit der Blutbehandlungsmaschine angepasst ist, einen zeitlichen Ausschnitt oder ein Zeitfenster aus dem zeitabhängigen Signal auszuschneiden, und wobei der Zeitpunkt der Anwendereingabe in dem zeitlichen Ausschnitt oder Zeitfenster oder benachbart dazu liegt.

5. Blutbehandlungssystem (300) nach einem der Ansprüche 1 bis 4, mit einer Eingabeeinheit (30) zur Eingabe der Anwendereingabe im Hinblick auf einen Betriebszustand oder einen Behandlungsverlauf oder auf eine Abweichung von einem idealen oder komplikationsfreien Betriebszustand der Blutbehandlungsmaschine oder einer Abweichung von einem idealen oder komplikationsfreien Behandlungsverlauf der Blutbehandlung.

6. Computerimplementiertes Verfahren (4000) zur Erkennung eines Betriebszustandes einer Blutbehandlungsmaschine oder eines Behandlungsverlaufs einer Blutbehandlung oder einer Abweichung von einem idealen oder komplikationsfreien Betriebszustand oder Behandlungsverlauf, umfassend:
Empfangen eines Betriebsparameterüberwachungssignals (401b), wobei das Betriebsparameterüberwachungssignal ein von einem zeitabhängigen Signal abgeleitetes Signal eines Sensors zur Überwachung eines Betriebsparameters der Blutbehandlungsmaschine darstellt, wobei eine Bildung des abgeleiteten Signals umfasst, dass ein zeitlicher Ausschnitt oder ein Zeitfenster aus dem zeitabhängigen Signal des Betriebsparameters ausgeschnitten wird,
Empfangen eines Anwenderreaktionssignals (402), wobei das Anwenderreaktionssignal ein Signal einer Anwendereingabe im Hinblick auf den Betriebszustand oder den Behandlungsverlauf oder die Abweichung von einem idealen oder komplikationsfreien Behandlungsverlauf oder Betriebszustand einer mit der Blutbehandlungsmaschine durchgeführten Blutbehandlung darstellt, wobei ein Zeitpunkt der Anwendereingabe in dem Ausschnitt oder dem Zeitfenster oder benachbart dazu liegt, und wobei das Anwenderreaktionssignal ein Anpassen einer Blutflussrate oder einer Ultrafiltrationsrate, eine Einleitung der Gabe eines Elektrolyts, ein Feststellen einer Blutdruckkrise, oder ein Feststellen einer Nadeldislokation, oder eine Auslösung eines Alarms oder eines Behandlungsstopps ist;
Abspeichern einer Mehrzahl von Anwenderreaktionssignalen und Betriebsparameterüberwachungssignalen in Bezug zueinander,
Erzeugen von Zuordnungs- oder Referenzdaten (411), die eine Zuordnung oder Korrelation eines bestimmten Anwenderreaktionssignals mit einem zugeordneten Betriebsparameterüberwachungssignal in dem Ausschnitt oder dem Zeitfenster herstellen,
Speichern der Zuordnungs- und Referenzdaten,
Empfangen eines bestimmten Betriebsparameterüberwachungssignals (422),
Abgleichen des bestimmten Betriebsparameterüberwachungssignals mit den Zuordnungs- oder Referenzdaten (412) um daraus eine typische Anwenderreaktion hierauf abzuleiten, und
Einleiten, durch eine Steuereinheit der Blutbehandlungsmaschine, einer Unterbrechung der Blutbehandlung als Antwort auf eine Abweichung einer typischen Anwenderreaktion an der die Steuereinheit aufweisenden Blutbehandlungsmaschine von der abgeleiteten typischen Anwenderreaktion, und/oder
Ausgeben über eine Benutzerschnittstelle eines Vorschlag an den Anwender, die abgeleitete typische Anwenderreaktion vorzunehmen, und/oder ein der abgeleiteten typischen Anwenderreaktion entsprechendes
automatisches Anpassen eines Betriebsparameter, und/oder Ausgeben an einer Ausgabeeinheit eines Alarms als Antwort auf eine Abweichung einer typischen Anwenderreaktion an einer der Blutbehandlungsmaschine (100, 201, 202, 20.., 20n) von der abgeleiteten typischen Anwenderreaktion.

7. Computerimplementiertes Verfahren (4000) nach Anspruch 6, wobei der Schritt des Erzeugens der Zuordnungs- oder Referenzdaten folgendes umfasst: Anwenden eines Lernalgorithmus, insbesondere eines neuronalen Netzes.

8. Computerimplementiertes Verfahren (4000) nach einem der Ansprüche 6 oder 7, enthaltend die folgenden Schritte:
Versehen des Anwendereingabesignals mit einem Zeitstempel, der den Zeitpunkt der Anwendereingabe in Bezug auf den Behandlungsverlauf angibt, bilden eines Zeitfensters oder eines zeitlichen Ausschnitts aus dem zeitabhängigen Signal, wobei der Zeitpunkt der Anwendereingabe in dem Zeitfenster oder dem zeitlichen Ausschnitt oder benachbart dazu liegt.

9. Computerprogrammprodukt umfassend Instruktionen, welche beim Laden in mindestens ein Speichermodul eines Computersystems (300) und Abarbeiten durch mindestens einen Prozessor des Computersystems, das computerimplementierte Verfahren (4000) nach einem der Ansprüche 6 bis 8 ausführen.

10. Computerprogramm umfassend Instruktionen zur Durchführung des computerimplementierten Verfahrens (4000) nach einem der Ansprüche 6 bis 8, wenn das Computerprogramm auf einem Computer ausgeführt wird.

## Claims

1. Blood treatment system (300) having at least one blood treatment machine (100, 201, 202, 20..., 20n) for conducting a blood treatment, having at least one sensor (211, 10, 21, 24, 25, 20, 27) for determining a time-dependent progression of an operating parameter during the blood treatment, and having a computer system for identifying an operational state of one of the blood treatment machines (100, 201, 202, 20..., 20n) or of a treatment course of a blood treatment or a deviation from an ideal or complication-free operational state or treatment course, wherein the computer system comprises:
a first interface (20, 312) adapted to receive an operational parameter monitoring signal, wherein the operational parameter monitoring signal represents a signal from the sensor for monitoring an operational parameter of the blood treatment machine (100, 201, 202, 20..., 20n) comprising the sensor, the signal being derived from a time-dependent signal and derived signal construction comprising a temporal portion or a time window being cut out of the time-dependent signal of the operational parameter,
a second interface (20, 312) adapted to receive a user reaction signal, wherein a user reaction signal represents a signal from a user input in view of the operational state or the treatment course or the deviation from an ideal or complication-free treatment course or operational state of a blood treatment conducted with the blood treatment machine of the blood treatment machines (100, 201, 202, 20..., 20n) comprising the sensor, the user input being found in the portion or time window or in an adjacent portion or time window to the portion or time window and the user reaction signal being an adjustment of a blood flow rate or an ultrafiltration rate, an initiation of electrolyte administration, a determination of a blood pressure crisis, or a determination of a needle dislocation, or a triggering of an alarm or a treatment stop;
an evaluation unit (40, 313) adapted to create assignment or reference data that establish a correlation of a specific user reaction signal with an assigned operational parameter monitoring signal,
a storage unit (311) configured to store the assignment and reference data,
wherein the evaluation unit (40, 313) is furthermore adapted to compare a specific operational parameter monitoring signal with the assignment or reference data in order to derive therefrom a typical user reaction thereto,
wherein one of the blood treatment machines (100, 201, 202, 20..., 20n) comprises a control unit (223) for initiating an interruption of the blood treatment in response to a deviation of a typical user reaction at the blood treatment machine comprising the control unit from the derived typical user reaction, and/or
wherein one of the blood treatment machines (100, 201, 202, 20..., 20n) is configured to output via a user interface a proposition for the user in relation to implementing the derived typical user reaction, and/or wherein one of the blood treatment machines (100, 201, 202, 20..., 20n) is configured to automatically implement an adjustment of an operational parameter corresponding to the derived typical user reaction, and/or
wherein the blood treatment system (300) comprises an output unit (212) for outputting an alarm in response to a deviation of a typical user reaction at one of the blood treatment machines (100, 201, 202, 20..., 20n) from the derived typical user reaction.

2. Blood treatment system (300) according to Claim 1, wherein the evaluation unit (40, 313) is adapted to apply a learning algorithm, in particular a neural network, during the creation of the assignment or reference data.

3. Blood treatment system (300) according to Claim 1, wherein the blood treatment machine (100, 201, 202, 20..., 20n) is a dialysis machine (100) and wherein the sensor (211, 10, 21, 24, 25, 20, 27) is selected from a blood temperature sensor (10, 21), a haematocrit sensor, a blood pressure sensor (20, 27), a conductivity sensor (24, 25) for determining a dialysate conductivity or a sensor for determining the power consumption of an actuator (23), preferably a blood pump (23).

4. Blood treatment system (300) according to any of Claims 1 to 3, wherein an evaluation unit (20) of the blood treatment machine (100, 201, 202, 20..., 20n) is adapted to provide the user input signal with a timestamp specifying the time of user input in relation to the treatment course, and wherein the evaluation unit of the computer system or the evaluation unit of the blood treatment machine is adapted to cut out a temporal portion or a time window from the time-dependent signal, and wherein the time of the user input is in the temporal portion or time window or adjacent thereto.

5. Blood treatment system (300) according to any of Claims 1 to 4, having an input unit (30) for inputting the user input in view of an operational state or a treatment course or in view of a deviation from an ideal or complication-free operational state of the blood treatment machine or a deviation from an ideal or complication-free treatment course of the blood treatment.

6. Computer- implemented method (4000) for identifying an operational state of a blood treatment machine or a treatment course of a blood treatment or a deviation from an ideal or complication-free operational state or treatment course, comprising:
receiving an operational parameter monitoring signal (401b), wherein the operational parameter monitoring signal represents a signal of a sensor for monitoring an operational parameter of the blood treatment machine the signal being derived from a time-dependent signal and derived signal construction comprising a temporal portion or a time window being cut out of the time-dependent signal of the operational parameter,
receiving a user reaction signal (402), wherein the user reaction signal represents a signal from a user input in view of the operational state or the treatment course or the deviation from an ideal or complication-free treatment course or operational state of a blood treatment conducted with the blood treatment machine, a user input time being found in the portion or time window or adjacent thereto and the user reaction signal being an adjustment of a blood flow rate or an ultrafiltration rate, an initiation of electrolyte administration, a determination of a blood pressure crisis, or a determination of a needle dislocation, or a triggering of an alarm or a treatment stop;
storing a plurality of user reaction signals and operational parameter monitoring signals in relation to one another,
creating assignment or reference data (411) that establish an assignment or correlation of a specific user reaction signal with an assigned operational parameter monitoring signal in the portion or the time window, storing the assignment and reference data,
receiving a specific operational parameter monitoring signal (422),
comparing the specific operational parameter monitoring signal with the assignment or reference data (412) in order to derive therefrom a typical user reaction thereto, and
by way of a control unit of the blood treatment machine initiating an interruption of the blood treatment in response to a deviation of a typical user reaction at the blood treatment machine comprising the control unit from the derived typical user reaction, and/or
outputting via a user interface a proposition for the user in relation to implementing the derived typical user reaction, and/or
automatically implementing an adjustment of an operational parameter corresponding to the derived typical user reaction, and/or
outputting on an output unit an alarm in response to a deviation of a typical user reaction at one of the blood treatment machines (100, 201, 202, 20..., 20n) from the derived typical user reaction.

7. Computer-implemented method (4000) according to Claim 6, wherein the step of creating the assignment or reference data comprises the following: applying a learning algorithm, in particular a neural network.

8. Computer-implemented method (4000) according to either of Claims 6 and 7, containing the following steps: providing the user input signal with a timestamp specifying the time of the user input in relation to the treatment course, forming a time window or a temporal portion from the time-dependent signal, wherein the time of the user input is found in the time window or the temporal portion or adjacent thereto.

9. Computer program product comprising instructions which when loaded into at least one storage module of a computer system (300) and worked through by at least one processor of the computer system execute the computer-implemented method (4000) according to any of Claims 6 to 8.

10. Computer program comprising instructions for carrying out the computer-implemented method (4000) according to any of Claims 6 to 8 when the computer program is executed on a computer.

## Revendications

1. Système de traitement du sang (300) comprenant au moins une machine de traitement du sang (100, 201, 202, 20..., 20n) destinée à effectuer un traitement du sang, au moins un capteur (211, 10, 21, 24, 25, 20, 27) destiné à déterminer l'évolution dans le temps d'un paramètre de fonctionnement pendant le traitement du sang, et un système informatique destiné à détecter un état de fonctionnement de l'une des machines de traitement du sang (100, 201, 202, 20..., 20n) ou une évolution d'un traitement du sang ou un écart par rapport à un état de fonctionnement ou déroulement de traitement idéal ou sans complication, le système informatique comprenant :
une première interface (20, 312) qui est adaptée pour recevoir un signal de surveillance de paramètre de fonctionnement, le signal de surveillance de paramètre de fonctionnement représentant un signal, qui est dérivé d'un signal dépendant du temps, du capteur destiné à surveiller un paramètre de fonctionnement de la machine de traitement du sang (100, 201, 202, 20..., 20n) comportant le capteur, la formation du signal dérivé comprenant la découpe d'une portion temporelle ou fenêtre temporelle dans le signal dépendant du temps du paramètre de fonctionnement,
une deuxième interface (20, 312) qui est adaptée pour recevoir un signal de réponse d'utilisateur, un signal de réponse d'utilisateur représentant un signal d'une entrée d'utilisateur concernant l'état de fonctionnement ou le déroulement du traitement ou l'écart par rapport à un déroulement de traitement ou à un état de fonctionnement idéal ou sans complication d'un traitement du sang effectué avec la machine de traitement du sang (100, 201, 202, 20..., 20n) comportant le capteur, l'entrée d'utilisateur étant située dans la portion ou fenêtre temporelle ou dans une portion ou fenêtre temporelle adjacente de la portion ou fenêtre temporelle, et le signal de réponse d'utilisateur étant un ajustement d'un débit sanguin ou d'un débit d'ultrafiltration, un début d'administration d'un électrolyte, une détection d'une crise de tension artérielle, ou une détection d'une dislocation d'aiguille, ou un déclenchement d'une alarme ou d'un arrêt du traitement ;
une unité d'évaluation (40, 313) qui est adaptée pour générer des données d'association ou de référence qui effectuent une corrélation entre un signal de réponse d'utilisateur déterminé et un signal de surveillance de paramètre de fonctionnement associé,
une unité de mémorisation (311) qui est configurée pour mémoriser les données d'association et de référence,
l'unité d'évaluation (40, 313) étant en outre adaptée pour comparer un signal de surveillance de paramètre de fonctionnement déterminé et les données d'association ou de référence afin d'en déduire une réaction d'utilisateur typique,
l'une des machines de traitement du sang (100, 201, 202, 20..., 20n) comportant une unité de commande (223) destinée à déclencher une interruption du traitement du sang en réponse à un écart entre une réaction d'utilisateur typique au niveau de la machine de traitement du sang comportant l'unité de commande et la réaction d'utilisateur typique dérivée, et/ou l'une des machines de traitement du sang (100, 201, 202, 20..., 20n) étant configurée pour délivrer à l'utilisateur, par le biais d'une interface utilisateur, une suggestion d'exécuter la réaction d'utilisateur typique dérivée, et/ou
l'une des machines de traitement du sang (100, 201, 202, 20..., 20n) étant configurée pour effectuer automatiquement un ajustement d'un paramètre de fonctionnement qui correspond à la réponse d'utilisateur typique, et/ou
le système de traitement du sang (300) comportant une unité de sortie (212) destinée à délivrer une alarme en réponse à un écart entre une réaction d'utilisateur typique au niveau d'une des machines de traitement du sang (100, 201, 202, 20..., 20n) et la réaction d'utilisateur typique.

2. Système de traitement du sang (300) selon la revendication 1, l'unité d'évaluation (40, 313) étant adaptée pour utiliser un algorithme d'apprentissage, notamment un réseau de neurones, lors de la génération des données d'association ou de référence.

3. Système de traitement du sang (300) selon la revendication 1, la machine de traitement du sang (100, 201, 202, 20..., 20n) étant une machine de dialyse (100), et le capteur (211, 10, 21, 24, 25, 20, 27) étant sélectionné parmi un capteur de température sanguine (10, 21), un capteur d'hématocrite, un capteur de pression artérielle (20, 27), un capteur de conductivité (24, 25) destiné à déterminer une conductivité d'un dialysat ou un capteur destiné à déterminer la consommation d'énergie d'un actionneur (23), de préférence une pompe sanguine (23).

4. Système de traitement du sang (300) selon l'une des revendications 1 à 3, une unité d'évaluation (20) de la machine de traitement du sang (100, 201, 202, 20..., 20n) étant adaptée pour fournir au signal d'entrée d'utilisateur un horodatage qui indique l'instant de l'entrée d'utilisateur par rapport au déroulement du traitement, et l'unité d'évaluation du système informatique ou l'unité d'évaluation de la machine de traitement du sang étant adaptée pour découper une portion temporelle ou une fenêtre temporelle dans le signal dépendant du temps, et l'instant de l'entrée d'utilisateur étant située dans la portion temporelle ou la fenêtre temporelle ou de manière adjacente à celle-ci.

5. Système de traitement du sang (300) selon l'une des revendications 1 à 4, comprenant une unité d'entrée (30) destinée à effectuer l'entrée d'utilisateur concernant un état de fonctionnement ou un déroulement de traitement ou un écart par rapport à un état de fonctionnement idéal ou sans complication de la machine de traitement du sang ou un écart par rapport à un déroulement idéal ou sans complication du traitement du sang.

6. Procédé informatisé (4000) de détection d'un état de fonctionnement d'une machine de traitement du sang ou d'un déroulement d'un traitement du sang ou d'un écart par rapport à un état de fonctionnement ou déroulement de traitement idéal ou sans complication, ledit procédé comprenant les étapes suivantes :
recevoir un signal de surveillance de paramètre de fonctionnement (401b), le signal de surveillance de paramètre de fonctionnement représentant un signal, dérivé d'un signal dépendant du temps, d'un capteur destiné à surveiller un paramètre de fonctionnement de la machine de traitement du sang, la formation du signal dérivé comprenant la découpe d'une portion temporelle ou d'une fenêtre temporelle dans le signal dépendant du temps du paramètre de fonctionnement ;
recevoir un signal de réponse d'utilisateur (402), le signal de réponse d'utilisateur étant un signal d'une entrée d'utilisateur par rapport à l'état de fonctionnement ou le déroulement du traitement ou l'écart par rapport à un déroulement de traitement ou un état de fonctionnement idéal ou sans complication d'un traitement du sang effectué avec la machine de traitement du sang, un instant de l'entrée d'utilisateur étant situé dans la portion ou la fenêtre temporelle ou de manière adjacente à celle-ci, et le signal de réponse d'utilisateur étant un ajustement d'un débit sanguin ou d'un débit d'ultrafiltration, un déclenchement d'administration d'un électrolyte, une détection d'une crise de tension artérielle, ou une détection d'une dislocation d'aiguille, ou un déclenchement d'une alarme ou d'un arrêt du traitement ;
mémoriser une pluralité de signaux de réponse d'utilisateur et de signaux de surveillance de paramètres de fonctionnement les uns par rapport aux autres,
générer des données d'association ou de référence (411), qui effectuent une association ou une corrélation entre un signal de réaction d'utilisateur déterminé et un signal de surveillance de paramètre de fonctionnement associé dans la portion ou la fenêtre temporelle,
mémoriser les données d'association et de référence,
recevoir un signal de surveillance de paramètre de fonctionnement déterminé (422),
comparer le signal de surveillance de paramètre de fonctionnement déterminé aux données d'association ou de référence (412) afin d'en dériver une réaction d'utilisateur typique, et
déclencher, par le biais d'une unité de commande de la machine de traitement du sang, une interruption du traitement du sang en réponse à un écart entre une réaction d'utilisateur typique au niveau de la machine de traitement du sang comportant l'unité de commande et
la réaction d'utilisateur typique dérivée, et/ou délivrer à l'utilisateur par le biais d'une interface utilisateur une suggestion d'effectuer la réaction d'utilisateur typique dérivée et/ou une adaptation automatique d'un paramètre de fonctionnement, laquelle correspond à la réaction d'utilisateur typique dérivée et/ou
délivrer une alarme au niveau d'une unité de sortie en réponse à un écart entre une réaction d'utilisateur typique au niveau de l'une des machines de traitement du sang (100, 201, 202, 20..., 20n) et la réaction d'utilisateur typique dérivée.

7. Procédé informatisé (4000) selon la revendication 6, l'étape de génération des données d'association ou de référence comprenant l'étape suivante :
appliquer un algorithme d'apprentissage, notamment un réseau neuronal.

8. Procédé informatisé (4000) selon l'une des revendications 6 ou 7, comprenant les étapes suivantes
doter le signal d'entrée d'utilisateur d'un horodatage qui indique l'instant de l'entrée d'utilisateur par rapport au déroulement du traitement,
former une fenêtre temporelle ou une portion temporelle à partir du signal dépendant du temps, l'instant de l'entrée d'utilisateur étant situé dans la fenêtre temporelle ou dans la portion temporelle ou de manière adjacente à celle-ci.

9. Produit de programme informatique comprenant des instructions qui, lorsqu'elles sont chargées dans au moins un module de mémorisation d'un système informatique (300) et traitées par au moins un processeur du système informatique, mettent en œuvre le procédé informatisé (4000) selon l'une des revendications 6 à 8.

10. Programme informatique comprenant des instructions destinées à mettre en œuvre le procédé informatisé (4000) selon l'une des revendications 6 à 8 lorsque le programme informatique est exécuté sur un ordinateur.
